# EUROPEAN PATENT SPECIFICATION

(11) **EP 3 280 381 B1**
(45) Date of publication and mention of the grant of the patent: **21.10.2020**
(21) Application number: 15713949.4
(22) Date of filing: 10.04.2015
(51) Int. Cl.: A61Q 9/02, A61K 8/02, A61K 8/34, A61K 8/36, A61K 8/67, A61K 8/81, A61K 8/92, B26B 21/44

(54) **SHAVING AID COMPOSITION**
RASIERHILFSMITTEL
COPOSITION DE RASAGE

(43) Date of publication of application: 14.02.2018
(73) Proprietor: BIC-Violex S.A., 145 69 Anixi, Attiki (GR)
(72) Inventor: BOUSVAROS, Gerasimos, 145 62 Kifissia (GR); ANTONIOU, Zoi, 190 01 Keratea-attica (GR)
(74) Representative: Peterreins Schley
(86) International application number: PCT/EP2015/057826
(87) International publication number: WO 2016/162080

(56) References cited:
- EP-A1- 1 125 697
- WO-A2-2012/051377
- WO-A2-2014/052390
- US-A1- 2012 216 408
- US-B2- 7 811 553

## Description

### FIELD OF THE INVENTION

The present invention relates to the field of shaving aid compositions. More specifically, the present invention relates to a shaving aid composition intended to be attached to a razor head as a shaving bar for an efficient and simplified shaving.

### BACKGROUND OF THE INVENTION

Conventional razors comprise a lubricating strip including a shaving aid composition which may not be sufficient to provide a comfortable shave by itself (i.e. without the use of an additional shaving aid).

In addition to conventional razors, there are in 1" razors comprising "gel bars" to eliminate need for an additional shaving aid, and razors with a skin conditioning solid which lather while shaving.

However, main problems with existing products involve either too bulky head size or a shaving aid formula which leaves an unpleasant sensation such a "slimy" residual on the skin for example. Also, a high dissolve rate in the shaving aid formula will limit the number of shaves before reaching its full design use life. On the other hand a low dissolve rate will not provide sufficient aid to the user in order to shave adequately without additional shaving aids during each shave.

There is therefore a need in the art for improved shaving aid compositions.

Incorporation of other functional components within the recommended range into shaving aid composition is thus often desirable for achieving an efficient shave.

A problem that the invention aims to solve is therefore to offer an improved shaving aid composition providing a comfortable, simplified and efficient shaving without the use of an additional shaving aid.

### SUMMARY OF THE INVENTION

For this purpose, an object of the invention is a shaving aid composition comprising:
- a glycol,
- an humectant including polyhydric alcohols selected from the group consisting of glycerin, sorbitol and mixture thereof,
- a soap base which is sodium and/or potassium cocoate, stearate, palmitate, myristate and mixtures thereof, and
- a surfactant being sodium laureth sulfate,
characterized in that it further comprises: about 0.5 to 4%, preferably 1.5 to 3.5% by weight of a film former wherein the film former is a mixture comprising hydrogenated cyclopentadiene, polyethylene, copernica cerifera wax and tocopherol, said percentage being calculated with respect to the total weight of the shaving aid composition.

Unless otherwise specified, all percentages and amounts expressed herein and elsewhere in the specification should be understood to refer to percentages by weight calculated with respect to the total weight of the shaving aid composition.

The invention is based in part on the finding that a shaving aid composition comprising notably about 0.5 to 4%, preferably 1.5 to 3.5% by weight of a film former can provide excellent shaving properties and skin conditioning benefits.

Indeed, the shaving aid composition according to the invention increases shaving quality and performance, by improving glideness, skin smoothness and skin conditioning.

Furthermore, the composition according to the invention reduces the slimy residue that is known in some existing products and has a right dissolve rate balance.

According to an embodiment, the shaving aid composition comprises about 0.5 to 5%, preferably about 0.5-0.8% by weight of an emollient and about 0.5 to 4%, preferably 1.5 to 3.5% by weight of a film former and about 0.5 to 4% by weight, preferably 2.5 to 3.5% of a multifunctional polymer.

### Emollient

The shaving aid composition according to the invention may include an emollient to bring softness, emolliency and spreadability to the composition.

According to an embodiment; the emollient is hydrogenated polydecene.

According to a particular embodiment; the emollient is hydrogenated polydecene, commercially available as Dekanex ® and is comprised in the shaving aid composition in an amount of 0.5 to 5%, preferably in an amount of 0.5 to 0.8%, and even more preferably in an amount of 0.7% by weight.

### Film former

The shaving aid composition according to the invention includes a film former to improve water resistance and rub resistance.

According to an embodiment, the film former is a mixture comprising hydrogenated polycyclopentadiene, polyethylene, copernica cerifera wax and tocopherol.

According to a particular embodiment, the film former is a mixture comprising hydrogenated polycyclopentadiene, polyethylene, copernica cerifera wax and tocopherol, commercially available as Koboguard HRPC ® and is comprised in an amount of 0.5 to 4%, preferably in an amount of 1.5 to 3.5%, and even more preferably in an amount of 2% by weight.

### Multifunctional polymer

The shaving aid composition according to the invention may include a multifunctional polymer to improve skin feel and foam aesthetics.

According to an embodiment, the multifunctional polymer is polypropylene terephthalate.

According to a particular embodiment; the multifunctional polymer is selected from the group consisting of polypropylene terephthalate, commercially available as Aristoflex PEA ® and is comprised in an amount of 0.5 to 4%, preferably in an amount of 2.5 to 3.5%, and even more preferably in an amount of 3% by weight.

### Glycol

According to the invention, the composition comprises a glycol which acts as a viscosity adjuster and/or as a solvent and/or as a skin conditioning agent.

According to an embodiment, the glycol is chosen in the group consisting of propylene glycol, ethylene glycol, diethylene glycol and mixtures thereof.

According to a particular embodiment, the composition comprises propylene glycol.

According to an embodiment, the composition comprises a glycol in an amount of 20-35% by weight, preferably in an amount of 24-30% by weight.

### Humectant

The shaving aid composition according to the invention includes a humectant to impart hydrating properties.

Humectant(s) may be used in an amount of about 30-50% by weight, preferably, in an amount of 33-38% by weight.

Humectants for use the present invention include polyhydric alcohols selected from the group consisting of glycerin, sorbitol and mixture thereof.

According to an embodiment, the shaving aid composition comprises a mixture of sorbitol and glycerin.

Humectants useful in the present invention can also include other components such as Aloe Vera powder, for example in an amount of 0.001-0.05% by weight and/or shea butter for example in an amount of 0.2-1% by weight.

According to a particular embodiment, the composition comprises a mixture of 15-25% by weight of sorbitol, preferably 15-18% by weight of sorbitol and 15-25% by weight of glycerin, preferably 16-20% by weight of glycerin. The high amount of glycerin gives notably comfort to the skin.

### Soap base

The soap base for use in the present invention is sodium and/or potassium cocoate, stearate, palmitate, myristate and mixtures thereof.

A particularly preferred soap base is a sodium stearate soap base.

According to an embodiment, the soap base is used in an amount of 15-24% by weight, preferably in an amount of 18-22% by weight.

### Surfactant

Surfactant(s) used in the present invention provide cleansing and emulsifying properties.

The surfactant used in the present invention is sodium laureth sulfate.

According to an embodiment, the surfactant is used in an amount of 6.5-12.5% by weight, preferably in an amount of 8-12% by weight.

### Other components

According to an embodiment, the shaving aid composition contains at least one additional component that is added to impart specific properties such as coloring agent, fragrance, chelating agent, opacifier, antibacterial agent or exfoliating particles.

The coloring agents are used in very low range, typically used in range from about 0.0001% to about 0.001% by weight.

Fragrance is also used in very low range, preferably no more than 0.5% by weight.

When the composition comprises a chelating agent, it is included in a range from 0.05% to 0.2% by weight. Different chelating agent, well known in the art in the cosmetic field, may be employed in the composition according to the invention, as for example Dissolvine GL47S ®.

When the composition comprises an opacifier, it is included in a range from 0.2% to 2% by weight. Different opacifier, well-known in the art in the cosmetic field, may be employed in the composition according to the invention, as for example Opulyn 301 ®.
According to a particular embodiment, the shaving aid composition of the present invention comprises:
- about 20-35% by weight, preferably 24-30% by weight of a glycol;
- about 30-50% by weight, preferably 33-38% by weight of an humectant
- about 15-24% by weight, preferably 18-22% by weight of a soap base,
- about 6.5-12.5% by weight, preferably 8-12% by weight of a surfactant,
- about 0.5-5% by weight, preferably 0.5-0.8% of hydrogenated polydecene,
- about 0.5-4% by weight, preferably 2.5-3.5% of polypropylene terephthalate; and
- about 0.5-4% by weight, preferably 1.5-3.5% of a mixture comprising hydrogenated polycyclopentadiene, polyethylene, copernica cerifera wax and tocopherol.
According to another particular embodiment, the shaving aid composition of the present invention comprises:
- about 20-35% by weight, preferably 24-30% by weight of propylene glycol;
- about 15-25% by weight, preferably 16-20% by weight of glycerin,
- about 15-25% by weight, preferably 15-18% by weight of sorbitol,
- about 15-24% by weight, preferably 18-22% by weight of sodium stearate,
- about 6.5-12.5% by weight, preferably 8-12% by weight of sodium laureath sulfate,
- about 0.5-5% by weight, preferably 0.5-0.8% of hydrogenated polydecene,
- about 0.5-4% by weight, preferably 2.5-3.5% of polypropylene terephthalate; and
- about 0.5-4% by weight, preferably 1.5-3.5% of a mixture comprising hydrogenated polycyclopentadiene, polyethylene, copernica cerifera wax and tocopherol.

The shaving aid composition according to the invention can be manufactured by the following method comprising the step consisting of:
(i) dissolving a film former in a first amount of glycol to form a first mixture;
(ii) preparing a second mixture comprising a second amount of glycol with an humectant;
(iii) adding the first mixture to the second mixture;
(iv) adding a surfactant;
(v) adding an emollient and a multifunctional polymer to the mixture obtained in step (iv); and
(vi) optionally adding additional components chosen in the group consisting of coloring agent, fragrance, chelating agent, opacifier, antibacterial agent or exfoliating particles to the mixture obtained in step (v).

Then, a shaving bar can be prepared from the shaving aid composition by process, well-known from the person skilled in the art, i.e by extrusion or by molding.

Another object of the present invention is a razor cartridge comprising:
- a housing having a front edge and a rear edge;
- one or more shaving blades between the front edge and the rear edge; and
- at least one shaving bar disposed in front of the blade(s) and/or at rear of the blade(s), the shaving bar being made of the shaving aid composition as defined previously.

According to an embodiment, the shaving bar is disposed in a shaving bar holder.

According to a particular embodiment, the razor cartridge comprises two shaving bars, one disposed in front of the blade(s) and another disposed at rear of the blade(s).

According to another embodiment, the razor cartridge comprises an additional strip, such as a lubricating strip disposed between the blade(s) and the shaving bar.

Another objet of the present invention is a razor comprising:
- an handle, and
- a razor cartridge as defined previously.

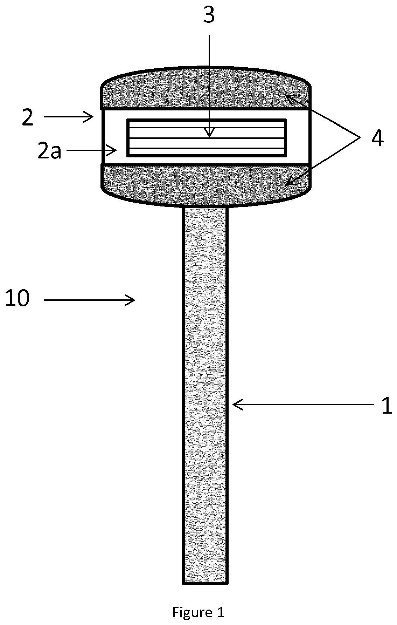

Figure 1 represents a razor including a razor cartridge, said razor cartridge comprising a shaving bar made of the shaving aid composition of the present invention.

The razor 10 of figure 1 comprises a handle 1 and a razor cartridge 2 having a housing 2a, said housing 2a including a front edge and a rear edge (not represented in figure 1).

Several blades 3 are disposed between the front edge and the rear edge of the housing 2a.

In this embodiment, a shaving bar 4 is disposed in front of the blades 3 and another shaving bar 4 is disposed at rear of the blades 3, the shaving bars 4 being made of shaving aid composition of the present invention.

When the shaving bar is in contact with water, foam is formed in contact with the skin providing an efficient and pleasant shaving without the need of an additional shaving aid.

Another object of the present invention is the use of a mixture comprising about 0.5 to 5% by weight of an emollient, and/or about 0.5 to 4% of a film former; and/or about 0.5 to 4% of a multifunctional polymer in a shaving aid composition for providing softness, emolliency and spreadability.

### EXAMPLE 1

The composition of the present invention was prepared by a hot mix of the components described in the table below.

**Table 1: shaving aid composition of example 1**

| **Components** | **Amount (%)w** |
|---|---|
| Propylene glycol | 20%-35% |
| Glycerin | 15%-25% |
| Sodium stearate | 15%-24% |
| Sorbitol | 15%-25% |
| Sodium Polyoxyethylene Laurylether Sulfate | 6.5%-12.5% |
| Hydrogenated Polycyclopentadiene | 1.6 |
| Polyethylene | |
| Copernicia Cerifera (Carnauba) Wax | |
| Tocopherol | |
| hydrogenated polydecene | 0,5 |
| Polypropylene Terephthalate | 2.5 |
| Opulyn 301 ® | 0.2%-2% |
| Shea butter | 0.2%-1% |
| Aloe vera powder | 0.001%-0.05% |
| Perfume | <0.5% |
| Dissolvine GL47S ® | 0.05%-0.2% |
| Color | 0.0001%-0.001% |

| | |
|---|---|
| where Opulyn ® 301 composition: Styrene/acrylic copolymer, Residual monomers, water. | |

## Claims

1. A shaving aid composition comprising:
a glycol,
an humectant, including polyhydric alcohols selected from the group consisting of glycerin, sorbitol and mixture thereof a soap base which is sodium and/or potassium cocoate, stearate, palmitate, myristate and mixtures thereof, and
a surfactant being sodium laureth sulfate,
**characterized in that** it further comprises:
about 0.5 to 4%, preferably 1.5 to 3.5% by weight of a film former wherein the film former is a mixture comprising hydrogenated polycyclopentadiene, polyethylene, copernica cerifera wax and tocopherol
said percentage being calculated with respect to the total weight of the shaving aid composition.

2. The shaving aid composition according to claim 1, wherein it further comprises:
about 0.5 to 5%, preferably about 0.5-0.8% by weight of an emollient with respect to the total weight of the shaving aid composition; and
about 0.5 to 4% by weight, preferably 2.5 to 3.5% of a multifunctional polymer;
said percentages being calculated with respect to the total weight of the shaving aid composition.

3. The shaving aid composition according to claim 2, wherein the emollient is hydrogenated polydecene.

4. The shaving aid composition according to claim 2, wherein the multifunctional polymer is polypropylene terephthalate.

5. The shaving aid composition according to claim 1, wherein the composition comprises a glycol in an amount of 20-35% by weight.

6. The shaving aid composition according to claim 5, wherein the glycol is propylene glycol.

7. The shaving aid composition according to any one of the preceding claims, wherein the composition comprises the humectant in an amount of 30-50% by weight.

8. The shaving aid composition according to any one of the preceding claims, wherein the composition comprises the surfactant in an amount of 6.5-12.5% by weight.

9. The shaving aid composition according to any one of the preceding claims comprising :
about 20-35% by weight, preferably 24-30% by weight of a glycol;
about 30-50% by weight, preferably 33-38% by weight of an humectant about 15-24% by weight, preferably 18-22% by weight of a soap base,
about 6.5-12.5% by weight, preferably 8-12% by weight of a surfactant,
about 0.5-5% by weight, preferably 0.5-0.8% of hydrogenated polydecene,
about 0.5-4% by weight, preferably 2.5-3.5% of polypropylene terephthalate ; and
about 0.5-4% by weight, preferably 1.5-3.5% of a mixture comprising hydrogenated polycyclopentadiene, polyethylene, copernica cerifera wax and tocopherol, said percentages being calculated with respect to the total weight of the shaving aid composition.

10. The shaving aid composition according to any one of the preceding claims comprising:
about 20-35% by weight, preferably 24-30% by weight of propylene glycol;
about 15-25% by weight, preferably 16-20% by weight of glycerin, about 15-25% by weight, preferably 15-18% by weight of sorbitol,
about 15-24% by weight, preferably 18-22% by weight of soap base wherein the soap base is sodium stearate,
about 6.5-12.5% by weight, preferably 8-12% by weight of sodium laureath sulfate,
about 0.5-5% by weight, preferably 0.5-0.8% of hydrogenated polydecene,
about 0.5-4% by weight, preferably 2.5-3.5% of polypropylene terephthalate;
about 0.5-4% by weight, preferably 1.5-3.5% of a mixture comprising hydrogenated polycyclopentadiene, polyethylene, copernica cerifera wax and tocopherol, said percentages being calculated with respect to the total weight of the shaving aid composition.

11. A razor cartridge comprising:
a housing having a front edge and a rear edge;
- one or more shaving blades between the front edge and the rear edge; and
at least one shaving bar disposed in front of the blade(s) and/or at rear of the blade(s), the shaving bar being made of the shaving aid composition according to any one of claims 1- 10.

## Patentansprüche

1. Rasiermittelzusammensetzung, die Folgendes umfasst:
ein Glykol,
ein Feuchthaltemittel, das mehrwertige Alkohole beinhaltet, die aus der Gruppe ausgewählt sind, die aus Glycerin, Sorbit und einer Mischung daraus besteht,
eine Seifenbasis, die Natrium- und/oder Kaliumsalz von Kokosfettsäuren, Stearat, Palmitat, Myristat und Mischungen daraus ist, und
ein Tensid, das Natriumlaurethsulfat ist,
**dadurch gekennzeichnet, dass** es ferner Folgendes umfasst:
etwa 0,5 bis 4 Gew.-%, vorzugsweise 1,5 bis 3,5 Gew.-%, eines Filmbildners, wobei der Filmbildner eine Mischung ist, die hydriertes Polycyclopentadien, Polyethylen, Carnaubawachs und Tocopherol umfasst, wobei der Prozentsatz hinsichtlich des Gesamtgewichts der Rasiermittelzusammensetzung berechnet wird.

2. Rasiermittelzusammensetzung nach Anspruch 1, wobei sie ferner Folgendes umfasst:
etwa 0,5 bis 5 Gew.-%, vorzugsweise etwa 0,5-0,8 Gew.-%, eines Weichmachers hinsichtlich des Gesamtgewichts der Rasiermittelzusammensetzung; und
etwa 0,5 bis 4 Gew.-%, vorzugsweise 2,5 bis 3,5 Gew.-%, eines multifunktionellen Polymers;
wobei die Prozentsätze hinsichtlich des Gesamtgewichts der Rasiermittelzusammensetzung berechnet werden.

3. Rasiermittelzusammensetzung nach Anspruch 2, wobei der Weichmacher hydriertes Polydecen ist.

4. Rasiermittelzusammensetzung nach Anspruch 2, wobei das multifunktionelle Polymer Polypropylenterephthalat ist.

5. Rasiermittelzusammensetzung nach Anspruch 1, wobei die Zusammensetzung ein Glykol in einer Menge von 20-35 Gew.-% umfasst.

6. Rasiermittelzusammensetzung nach Anspruch 5, wobei das Glykol Propylenglykol ist.

7. Rasiermittelzusammensetzung nach einem der vorhergehenden Ansprüche, wobei die Zusammensetzung das Feuchthaltemittel in einer Menge von 30-50 Gew.-% umfasst.

8. Rasiermittelzusammensetzung nach einem der vorhergehenden Ansprüche, wobei die Zusammensetzung das Tensid in einer Menge von 6,5-12,5 Gew.-% umfasst.

9. Rasiermittelzusammensetzung nach einem der vorhergehenden Ansprüche, die Folgendes umfasst:
etwa 20-35 Gew.-%, vorzugsweise 24-30 Gew.-%, eines Glykols;
etwa 30-50 Gew.-%, vorzugsweise 33-38 Gew.-%, eines Feuchthaltemittels,
etwa 15-24 Gew.-%, vorzugsweise 18-22 Gew.-%, einer Seifenbasis,
etwa 6,5-12,5 Gew.-%, vorzugsweise 8-12 Gew.-% eines Tensids,
etwa 0,5-5 Gew.-%, vorzugsweise 0,5-0,8 Gew.-% hydriertes Polydecen,
etwa 0,5-4 Gew.-%, vorzugsweise 2,5-3,5 Gew.-% Polypropylenterephthalat; und
etwa 0,5-4 Gew.-%, vorzugsweise 1,5-3,5 Gew.-% einer Mischung, die hydriertes Polycyclopentadien, Polyethylen, Carnaubawachs und Tocopherol umfasst, wobei die Prozentsätze hinsichtlich des Gesamtgewichts der Rasiermittelzusammensetzung berechnet werden.

10. Rasiermittelzusammensetzung nach einem der vorhergehenden Ansprüche, die Folgendes umfasst:
etwa 20-35 Gew.-%, vorzugsweise 24-30 Gew.-% Propylenglykol;
etwa 15-25 Gew.-%, vorzugsweise 16-20 Gew.-% Glycerin,
etwa 15-25 Gew.-%, vorzugsweise 15-18 Gew.-% Sorbit, etwa 15-24 Gew.-%, vorzugsweise 18-22 Gew.-% Gewicht von Seifenbasis, wobei die Seifenbasis Natriumstearat ist,
etwa 6,5-12,5 Gew.-%, vorzugsweise 8-12 Gew.-% Natriumlaurethsulfat,
etwa 0,5-5 Gew.-%, vorzugsweise 0,5-0,8 Gew.-% hydriertes Polydecen,
etwa 0,5-4 Gew.-%, vorzugsweise 2,5-3,5 Gew.-% Polypropylenterephthalat;
etwa 0,5-4 Gew.-%, vorzugsweise 1,5-3,5 Gew.-% einer Mischung, die hydriertes Polycyclopentadien, Polyethylen, Carnaubawachs und Tocopherol umfasst, wobei die Prozentsätze hinsichtlich des Gesamtgewichts der Rasiermittelzusammensetzung berechnet werden.

11. Rasierklingeneinheit, die Folgendes umfasst:
ein Gehäuse, das eine Vorderkante und eine Hinterkante aufweist;
- eine oder mehrere Rasierklingen zwischen der Vorderkante und der Hinterkante; und
wenigstens ein Rasier-Gleitstreifen, der vor der/den Klinge(n) und/oder hinter der/den Klinge(n) angeordnet ist, wobei der Rasier-Gleitstreifen aus der Rasiermittelzusammensetzung nach einem der Ansprüche 1-10 hergestellt ist.

## Revendications

1. Composition d'aide au rasage comprenant :
un glycol, un humectant, y compris des alcools polyhydriques choisis dans le groupe constitué par de la glycérine, du sorbitol et leur mélange, une base de savon qui est du cocoate de sodium et/ou de potassium, du stéarate, du palmitate, du myristate et leurs mélanges, et un tensioactif étant du laureth sulfate de sodium, **caractérisée en ce qu'**elle comprend en outre :
environ 0,5 à 4 %, de préférence 1,5 à 3,5 % en poids d'un filmogène, le filmogène étant un mélange comprenant du polycyclopentadiène hydrogéné, du polyéthylène, de la cire de copernica cerifera et du tocophérol, ledit pourcentage étant calculé par rapport au poids total de la composition d'aide au rasage.

2. Composition d'aide au rasage selon la revendication 1, comprenant en outre :
environ 0,5 à 5 %, de préférence environ 0,5 à 0,8 % en poids d'un émollient par rapport au poids total de la composition d'aide au rasage ; et
environ 0,5 à 4 % en poids, de préférence 2,5 à 3,5 % d'un polymère multifonctionnel ;
lesdits pourcentages étant calculés par rapport au poids total de la composition d'aide au rasage.

3. Composition d'aide au rasage selon la revendication 2, dans laquelle l'émollient est du polydécène hydrogéné.

4. Composition d'aide au rasage selon la revendication 2, dans laquelle le polymère multifonctionnel est le polytéréphtalate d'éthylène.

5. Composition d'aide au rasage selon la revendication 1, dans laquelle la composition comprend un glycol en une quantité de 20 à 35 % en poids.

6. Composition d'aide au rasage selon la revendication 5, dans laquelle le glycol est le propylèneglycol.

7. Composition d'aide au rasage selon l'une quelconque des revendications précédentes, dans laquelle la composition comprend l'humectant en une quantité de 30 à 50 % en poids.

8. Composition d'aide au rasage selon l'une quelconque des revendications précédentes, dans laquelle la composition comprend le tensioactif en une quantité de 6,5 à 12,5 % en poids.

9. Composition d'aide au rasage selon l'une quelconque des revendications précédentes, comprenant :
environ 20 à 35 % en poids, de préférence 24 à 30 % en poids d'un glycol ;
environ 30 à 50 % en poids, de préférence 33 à 38 % en poids d'un humectant, environ 15 à 24 % en poids, de préférence 18 à 22 % en poids d'une base de savon, environ 6,5 à 12,5 % en poids, de préférence 8 à 12 % en poids d'un tensioactif, environ 0,5 à 5 % en poids, de préférence 0,5 à 0,8 % de polydécène hydrogéné, environ 0,5 à 4 % en poids, de préférence 2,5 à 3,5 % de polytéréphtalate d'éthylène ; et
environ 0,5 à 4 % en poids, de préférence 1,5 à 3,5 % d'un mélange comprenant du polycyclopentadiène hydrogéné, du polyéthylène, de la cire de copernica cerifera et du tocophérol, lesdits pourcentages étant calculés par rapport au poids total de la composition d'aide au rasage.

10. Composition d'aide au rasage selon l'une quelconque des revendications précédentes, comprenant :
environ 20 à 35 % en poids, de préférence 24 à 30 % en poids de propylèneglycol ;
environ 15 à 25 % en poids, de préférence 16 à 20 % en poids de glycérine, environ 15 à 25 % en poids, de préférence 15 à 18 % en poids de sorbitol, environ 15 à 24 % en poids, de préférence 18 à 22 % en poids d'une base de savon, la base de savon étant le stéarate de sodium, environ 6,5 à 12,5 % en poids, de préférence 8 à 12 % en poids de laureth sulfate de sodium, environ 0,5 à 5 % en poids, de préférence 0,5 à 0,8 % de polydécène hydrogéné, environ 0,5 à 4 % en poids, de préférence 2,5 à 3,5 % de polytéréphtalate d'éthylène ;
environ 0,5 à 4 % en poids, de préférence 1,5 à 3,5 % d'un mélange comprenant du polycyclopentadiène hydrogéné, du polyéthylène, de la cire de copernica cerifera et du tocophérol, lesdits pourcentages étant calculés par rapport au poids total de la composition d'aide au rasage.

11. Cartouche de rasoir comprenant :
un boîtier ayant un bord avant et un bord arrière ;
- une ou plusieurs lames de rasage entre le bord avant et le bord arrière ; et
au moins une bande de rasage disposée devant la ou les lames et/ou à l'arrière de la ou des lames, la bande de rasage étant constituée de la composition d'aide au rasage selon l'une quelconque des revendications 1 à 10.
